(19) Europäisches Patentamt
European Patent Office
Office européen des brevets

(11) **EP 1 200 020 B1**

(12) **FASCICULE DE BREVET EUROPEEN**

(45) Date de publication et mention
de la délivrance du brevet:
**30.07.2008 Bulletin 2008/31**

(21) Numéro de dépôt: **00956581.3**

(22) Date de dépôt: **31.07.2000**

(51) Int Cl.:
***A61F 2/16*** *(2006.01)*

(86) Numéro de dépôt international:
**PCT/FR2000/002203**

(87) Numéro de publication internationale:
**WO 2001/008605 (08.02.2001 Gazette 2001/06)**

(54) **IMPLANT FORMANT LENTILLE INTRAOCULAIRE MULTIFOCALE**

MULTIFOKALES INTRAOCULARLINSENIMPLANTAT

IMPLANT FORMING MULTIFOCAL INTRAOCULAR LENS

(84) Etats contractants désignés:
**AT BE CH CY DE DK ES FI FR GB GR IE IT LI LU MC NL PT SE**

(30) Priorité: **30.07.1999 FR 9909941**

(43) Date de publication de la demande:
**02.05.2002 Bulletin 2002/18**

(73) Titulaire: **Ioltechnologie-Production**
**17180 Perigny (FR)**

(72) Inventeur: **BOSC, Jean-Michel**
**F-44220 Coueron (FR)**

(74) Mandataire: **Santarelli**
**14, avenue de la Grande Armée,**
**B.P. 237**
**75822 Paris Cedex 17 (FR)**

(56) Documents cités:
**WO-A-91/13597**       **WO-A-94/07435**
**WO-A-97/26842**       **US-A- 5 222 981**

**Description**

La présente invention concerne un implant formant lentille intraoculaire multifocale.

Elle s'inscrit de manière générale dans le domaine de l'implantation de lentilles intraoculaires dans le segment antérieur comprenant pour l'essentiel les chambres antérieure et postérieure de l'oeil pour corriger l'aphakie, c'est-à-dire l'absence du cristallin, notamment après l'extraction d'un cristallin atteint de cataracte.

Les implants intraoculaires sont utilisés pour remédier aux troubles de la réfraction générés par l'absence de cristallin.

L'implantation d'une lentille intraoculaire monofocale est couramment réalisée pour remplacer le cristallin. Classiquement, des éléments haptiques s'étendent à la périphérie de l'élément optique et sont adaptés à venir en appui dans le sac capsulaire, entre les capsules antérieures et postérieures, ou éventuellement dans le sulcus ciliaire.

Cependant, ce type d'implant ne permet pas de corriger des troubles de la vue, tels que l'astigmatisme, l'hypermétropie ou la presbytie induite par l'extraction de la cataracte.

On a tenté d'implanter des lentilles intraoculaires multifocales, comportant un élément optique qui présente des corrections différentes, éventuellement progressives, entre son centre et sa périphérie.

Cependant, il est difficile de prédire la correction exacte qu'il convient d'appliquer à l'oeil et la satisfaction du patient ne peut être garantie totalement.

Les échecs sont dus en particulier à des erreurs de biométrie, à l'apparition d'astigmatisme, ou encore à des facteurs provenant du patient lui-même, par exemple une pathologie neuro-visuelle associée à un facteur psychologique où la multifocalité est mal supportée).

Actuellement, après implantation monofocale, le patient doit en outre porter une correction (des lunettes ou des verres de contact) pour corriger les troubles de la vue : perte d'accommodation, amétropie résiduelle, astigmatisme induits.

En cas de troubles importants, il est parfois nécessaire de procéder à une nouvelle intervention chirurgicale portant sur la cornée par chirurgie réfractive ou par enlèvement de l'implant, par changement de celui-ci rajout d'un autre implant devant le premier implant, c'est-à-dire à l'antérieure, soit dans le sac capsulaire, soit dans le sulcus ciliaire, voire la chambre antérieure ou fixé à l'iris.

On connaît également une technique de double implantation de lentilles intraoculaires du document US-A-5.769.890, en première implantation dans le sac capsulaire et en seconde implantation dans le sulcus ciliaire. La seconde lentille est censée corriger des défauts associés à la première lentille. Mais le seul exemple pratique donné concerne la correction de l'astigmatisme par la rotation de la seconde lentille par rapport à la première qui a une puissance dioptrique dans un quadrant différent. A cette fin, les éléments haptiques des lentilles respectives sont disposés sensiblement perpendiculairement les uns aux autres. Enfin, il n'est nullement question de correction de la vision de près et de la vision de loin.

Le document WO 94/07435 décrit la technique de la double implantation de lentille intraoculaire où la seconde lentille est censée corriger les défauts résiduels de la vue après cicatrisation suite à l'implantation de la première lentille. Pour la seconde implantation, il est envisagé également des lentilles de faible dioptrie. Il décrit également le remplacement d'une optique multifocale par une autre optique multifocale ou monofocale lorsque la première optique multifocale n'a pas été tolérée.

La présente invention a pour but de proposer un implant intraoculaire multifocal qui puisse corriger de manière efficace à la fois les troubles de la réfraction et la presbytie induite lorsque le praticien peut avoir connaissance des éléments nécessaires pour corriger la vue, et ce sans risque d'affecter la position ou l'orientation d'une lentille principale pré-implantée dans le sac capsulaire.

Ainsi, l'implant formant lentille intraoculaire multifocale visé par l'invention comprend un élément optique et des éléments haptiques s'étendant à la périphérie de l'élément optique.

Conformément à l'invention, l'élément optique forme une lentille multifocale de basse puissance destinée à corriger une éventuelle amétropie résiduelle et la vision de près.

Ainsi, la lentille multifocale peut être implantée de manière optimale, en addition d'un premier implant implanté dans le sac capsulaire du segment antérieur comprenant notamment les chambres antérieure et postérieure de l'oeil.

Il est en effet possible de calculer de manière précise la correction multifocale qu'il est nécessaire d'introduire dans l'oeil après la mise en place d'un premier implant et sa stabilisation dans la chambre postérieure de l'oeil.

Le fonctionnement multifocal de la lentille et sa puissance peuvent ainsi être prédits de manière fiable, à partir par exemple d'essais de correction par lentille de contact multifocale.

Selon la présente invention est pourvu un ensemble d'implants intraoculaires pour segment antérieur de l'oeil, comprenant une lentille intraoculaire principale comportant un élément optique et des éléments haptiques s'étendant à la périphérie de l'élément optique, la lentille intraoculaire principale étant conformée pour implantation dans le sac capsulaire et une lentille secondaire comportant des éléments haptiques, un élément optique adapté à apporter une correction d'une amétropie résiduelle résultant de la lentille principale, l'élément optique de la lentille intraoculaire secondaire étant multifocale et adapté à corriger la vision de près, caractérisé en ce que la lentille intraoculaire secondaire est conformée pour implantation dans le sulcus ciliaire.

Selon une caractéristique préférée de l'invention, l'implant principal est un implant formant lentille intraoculaire monofocale adaptée à remplacer le cristallin. L'implant formant lentille intraoculaire multifocale permet ainsi d'une part de corriger l'amétropie résiduelle et d'autre part, d'obtenir une vision multifocale, c'est-à-dire une bonne vision de loin et de près.

Selon une caractéristique préférée de l'invention, l'élément optique forme une lentille multifocale et permet de corriger l'éventuelle amétropie résiduelle existant après mise en place et stabilisation de l'implant principal pré-implanté.

Ainsi, l'élément optique forme de préférence une lentille multifocale de puissance de base de +5 dioptries pour corriger l'amétropie résiduelle et d'une puissance supplémentaire pour corriger la vision de près comprise entre +2 et +5 dioptries.

De manière analogue, selon la présente invention, est pourvu également un implant formant lentille intraoculaire multifocale comprenant un élément optique apte à corriger l'amétropie résiduelle résultant d'un implant principal et des éléments haptiques **caractérisés en ce que** l'implant est conformé pour l'implantation dans le sulcus ciliaire.

D'autres particularités et avantages de l'invention apparaîtront encore dans la description ci-après.

Aux dessins annexés, donnés à titre d'exemples non limitatifs :

- la figure 1 est une vue de face d'un implant formant lentille multifocale conforme à un premier mode de réalisation de l'invention ;
- la figure 2 est une vue de côté de l'implant de la figure 1; et
- la figure 3 est une vue en coupe d'un oeil illustrant l'implantation de l'implant de la figure 1.

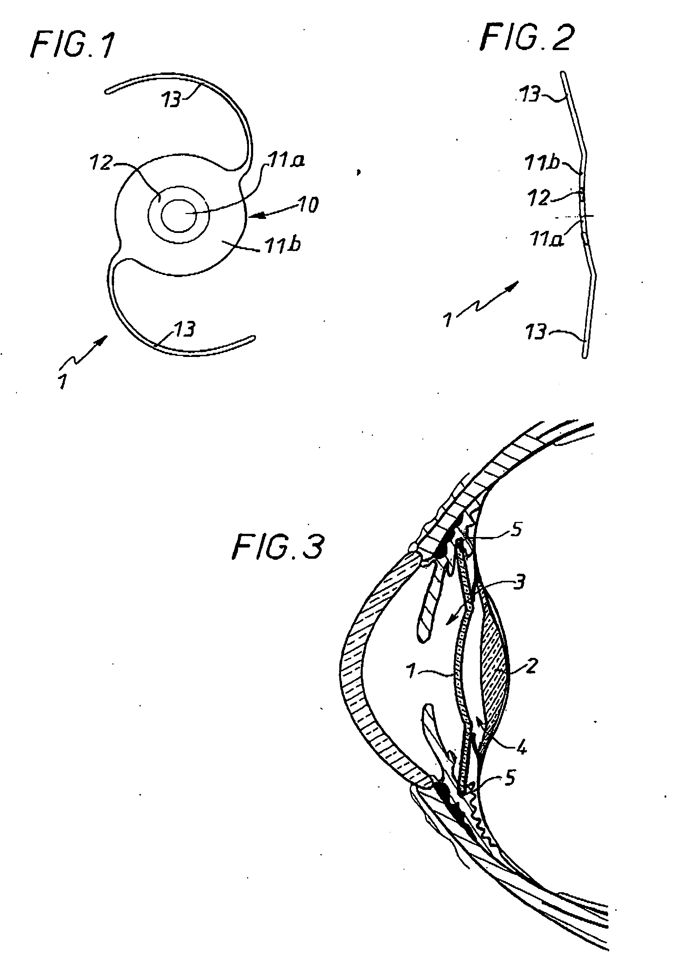

On va décrire tout d'abord la structure d'un implant multifocal conforme au mode de réalisation illustré aux figure 1 et 2.

Cet implant 1 forme une lentille intraoculaire multifocale adaptée à corriger une éventuelle amétropie résiduelle résultant d'un implant principal pré-implanté, décrit dans la suite de la description et à ajouter une correction pour obtenir une vision nette de près.

Cet implant multifocal 1 comprend un élément optique 10 et des éléments haptiques 13 qui s'étendent à la périphérie de l'élément optique 10.

Dans cet exemple, l'élément optique 10, de forme générale circulaire, comprend une portion centrale 11a et une portion annulaire périphérique 11b ayant une puissance comprise par exemple entre -10 et +10 dioptries et une portion annulaire intermédiaire 12 ayant une puissance supplémentaire comprise entre 2 et 5 dioptries.

L'élément optique 10 forme ainsi une lentille multifocale de basse puissance. Elles correspondent à une correction chez le myope fort pour deux raisons : imprécision du calcul biométrique et état rétinien central des myopes forts. De telles lentilles n'ont pas été adoptées à ce jour.

L'une et/ou l'autre face de l'élément optique 10 comportent ainsi des zones à différents rayons de courbure permettant d'obtenir une lentille multifocale par réfraction.

Les éléments haptiques 13 de l'implant multifocal 1 sont constitués dans cet exemple d'anses 13 qui s'étendent dans le même sens (par exemple dans le sens inverse des aiguilles d'une montre dans la vue de face à la figure 1) à partir de l'élément optique 10. Ces anses 13 sont diamétralement opposées.

Il va de soi que d'autres formes d'haptiques sont possibles, telles que des haptiques plates.

Cet implant multifocal 1 est placé en position antérieure comme illustré à la figure 3, dans la chambre postérieure 3 de l'oeil alors qu'un implant principal 2, implanté en premier, est en position postérieure.

Cet implant principal 2 est dans cet exemple un implant standard formant lentille intraoculaire monofocale.

Il est implanté dans le sac capsulaire 4 et vient en appui entre la capsule antérieure et la capsule postérieure du sac capsulaire 9.

Cet implant principal 2 forme une lentille intraoculaire monofocale adaptée à remplacer le cristallin de manière optimum et efficace.

L'implant multifocal 1 conforme à ce mode de réalisation de l'invention est alors implanté dans le segment antérieur, à savoir dans le sulcus ciliaire 5.

Ces deux implants 1, 2 peuvent être en tout type de matériau pourvu que leur stabilité soit assurée.

En particulier, des anses rigides 13 offrent une meilleure stabilité à l'implant multifocal 1 pour son implantation dans le sulcus ciliaire.

Toute configuration d'optique de l'implant multifocal est possible, telle que ménisque.

Dans un tel mode de réalisation, l'implant multifocal 2 choisi est de forme ménisquée antérieure (c'est-à-dire concave sur sa face postérieure et convexe sur sa face antérieure).

En outre, dans ce mode de réalisation tel qu'illustré à la figure 2, les anses 13 de l'implant multifocal 1 sont inclinées antérieurement, selon une inclinaison judicieusement choisie pour éviter tout risque de contact de cet implant 1 avec l'iris.

On va décrire à présent le procédé d'implantation double des implants principal 2 et multifocal 1 décrits précédemment.

On met en place tout d'abord l'implant principal 2, formant ici une lentille monofocale, par chirurgie classique, les dimensions des éléments haptiques 21 leur permettant de venir en appui élastiquement dans le sac capsulaire.

On analyse ensuite le résultat de cette première implantation en recherchant notamment l'amétropie résiduelle et les erreurs d'astigmatisme.

On procède ainsi à des essais par lentille cornéenne d'une correction multifocale pour déterminer la correction à appliquer en fonction des troubles existants.

Le calcul de la puissance de base de la lentille multifocale en fonction de l'amétropie résiduelle du premier implant peut être obtenu de différentes manières.

Après une première implantation dans le sac capsulaire, un patient a une réfraction post-opératoire (amétropie résiduelle) stabilisée à une valeur que l'on appelle $R_{post}$. Il s'agit ici de déterminer la formule à appliquer pour calculer la valeur de la puissance de base de la lentille multifocale.

On néglige l'épaisseur de la lentille de deuxième implantation. Sa puissance étant relativement faible (inférieure à +10D), l'erreur engendrée est négligeable.

Les notations utilisées sont les suivantes :

$P_{post}$ : puissance totale de l'oeil en post opératoire de la première implantation

$R_{post}$ : réfraction en post opératoire de la première implantation

$P_E$ : puissance emmétropisante de l'oeil (calculée après la première implantation)

e : distance de la cornée à la lentille de correction (en m)

e' : distance de la première à la deuxième lentille (en m)

K : kératométrie

ACD : profondeur de chambre antérieure (en m) = e + e'

n : indice de l'humeur aqueuse (1.336)

$P_{IOL}$ : puissance de la lentille de première implantation

$P_{IOLC}$ : puissance de la lentille de correction

Formule optique théorique complète:

La puissance de l'oeil en post-opératoire de la première implantation est :

$$P_{post} = K + P_{IOL} - ACD/n \cdot (K \cdot P_{IOL}) \qquad (1)$$

la puissance emmétropisante de l'oeil est la puissance actuelle corrigée de l'amétropie mesurée :

$$P_E = P_{post} + R_{post} \qquad (2)$$

$$P_E = K + P_{IOL} - ACD/n \cdot (K \times P_{IOL}) + R_{post} \qquad (3)$$

en appliquant la formule de Gullstrandt sur les dioptres de la cornée et des lentilles de première et deuxième intention, on obtient :

$$P_E = K + (P_{IOLC} + P_{IOL} - e'/n \, P_{IOLC} \cdot P_{IOL}) - e/n \, K(P_{IOLC} + P_{IOL} - e'/n \, P_{IOLC} \cdot P_{IOL}) \qquad (4)$$

en remplaçant $P_E$ par (3), il vient (5),

$$K + P_{IOL} - ACD/n \cdot (K \times P_{IOL}) + R_{post} - K - P_{IOL} + (e/n \cdot K \cdot P_{IOL}) = P_{IOLC} - (e'/n \cdot P_{IOLC} \cdot P_{IOL}) - (e/n \cdot K \cdot P_{IOLC}) + (ee'/n^2 \cdot K \cdot P_{IOL} \cdot P_{IOLC}) \quad (5)$$

d'où la puissance de correction en sulcus

$$P_{IOLC} = (R_{post} - e'/n \cdot K \cdot P_{IOL}) / (1 - e/n \cdot K - e'/n \cdot P_{IOL} + ee'/n^2 \cdot K \cdot P_{IOL}) \quad (6)$$

Formule optique théorique simplifiée :
Si l'on considère e' (distance entre les 2 implants) suffisamment petit pour négliger tous les termes en e' dans (6), il résulte :

$$P_{IOLC} \simeq R_{post} / (1 - e/n \cdot K) \quad (7)$$

Si l'on prend un oeil aux biométries standards, le terme (1 -e/n . K) est à peu près constant. D'où la règle approximative dite des 1.25 :

$$P_{IOLC} \simeq 1.25 \cdot R_{post} \quad (8)$$

Deux exemples de calculs :

1) Premier implant de 23.5 D, amétropie résiduelle de -2D

L = 24.01 mm
K = 43.08 D
ACD = 0.005 m
$P_{IOL}$ = 23.5 D
$P_E$ = 60.69 D
$R_{post}$ = -2.1 D

si e' = 1mm (e = 4.0) :
$P_{IOLC}$ = -3.3 avec la formule complète (6)
$P_{IOLC}$ = -2.4 avec la formule simplifiée (7)
$P_{IOLC}$ = -2.6 avec la règle de 1.25 (8)

si e' = 0.5mm (e = 4.5) :
$P_{IOLC}$ = -2.9 avec la formule complète (6)
$P_{IOLC}$ = -2.45 avec la formule simplifiée (7)
$P_{IOLC}$ = -2.6 avec la règle de 1.25 (8)

2) Premier implant de 18.4 D, amétropie résiduelle de -2D

L = 24.01 mm
K = 43.08 D
ACD = 0.005 m
$P_{IOL}$ = 18.4 D
$P_E$ = 56.41 D
$R_{post}$ = -2.1 D

si e' = 1mm :
$P_{IOLC}$ = -3.1 avec la formule complète (6)
$P_{IOLC}$ = -2.4 avec la formule simplifiée (7)
$P_{IOLC}$ = -2.6 avec la règle de 1.25 (8)

si e' = 0.5mm
$P_{IOLC}$ = -2.8 avec la formule complète (6)
$P_{IOLC}$ = -2.45 avec la formule simplifiée (7)
$P_{IOLC}$ = -2.6 avec la règle de 1.25 (8)

Plus la distance entre les lentilles est grande, plus l'écart est important entre les résultats des diverses formules.

De même avec la puissance de l'implant, plus elle est importante, plus un écart est observé entre les différentes formules.

D'autres formules peuvent être définies.

On réalise ensuite une deuxième opération pour monter la lentille multifocale ayant une puissance faible, calculée selon les formules précédentes à partir de l'amétropie résiduelle, et un fonctionnement multifocal déterminé à partir de l'essai effectué par lentilles cornéennes.

Cet implant multifocal 1 est posé indépendamment de l'implant monofocal 2, dans le sulcus ciliaire dans la chambre antérieure.

La faible puissance de l'élément optique 10 de l'implant multifocal 1 est rendue possible du fait qu'il est monté en complément d'un premier implant 2 qui corrige en première approximation l'absence de cristallin.

Cet implant multifocal 1 peut également permettre de corriger l'astigmatisme résiduel par la forme torique de l'élément optique, c'est-à-dire un élément à la fois torique et multifocal.

Bien entendu, de nombreuses modifications peuvent être apportées à l'exemple de réalisation décrit ci-dessus sans sortir du cadre de l'invention.

Ainsi, l'implant multifocal 1, décrit dans les exemples ci-dessus comme un implant réfractif, pourrait également avoir un effet multifocal obtenu par diffraction ou autre.

## Revendications

1. Ensemble d'implants intraoculaires pour segment antérieur de l'oeil, comprenant une lentille intraoculaire principale (2) comportant un élément optique et des éléments haptiques s'étendant à la périphérie de l'élément optique, la lentille intraoculaire principale étant conformée pour implantation dans le sac capsulaire (4) et une lentille secondaire (1) comportant des éléments haptiques (13), un élément optique (10) adapté à apporter une correction d'une amétropie résiduelle résultant de la lentille principale, l'élément optique de la lentille intraoculaire secondaire étant multifocal (11a, 11 b) et adapté à corriger la vision de près, **caractérisé en ce que** la lentille intraoculaire secondaire (1) est conformée pour implantation dans le sulcus ciliaire (5).

2. Ensemble d'implants conforme à la revendication 1, **caractérisé en ce que** la lentille intraoculaire principale (2) est monofocale (2) et adaptée à remplacer optiquement le cristallin.

3. Implant conforme à l'une des revendications 1 ou 2, **caractérisé en ce que** l'élément optique (10) forme une lentille multifocale de puissance de base de-10 à +10 dioptries pour corriger l'amétropie résiduelle et d'une puissance supplémentaire pour la vision de près comprise entre +2 et +5 dioptries.

4. Implant formant lentille intraoculaire multifocale comprenant un élément optique (10) apte à corriger l'amétropie résiduelle résultant d'un implant principal et des éléments haptiques (13) **caractérisés en ce que** l'implant est conformé pour l'implantation dans le sulcus ciliaire (5).

## Claims

1. Set of intraocular implants for the anterior segment of the eye, comprising a main intraocular lens (2) comprising an optical element and haptic elements lying at the periphery of the optical element, the main intraocular lens being conformed for implantation in the capsular sac (4), and a secondary lens (1) comprising haptic elements (13), an optical element (10) adapted to afford a correction of residual ametropia resulting from the main lens, the optical element of the secondary intraocular lens is multifocal (11a, 11 b) and for correcting close vision, **characterised in**

**that** the secondary intraocular lens (1) is conformed for implantation in the ciliary sulcus (5).

2. Set of implants according to claim 1, **characterised in that** the main intraocular lens (2) is monofocal (2) and adapted to optically replace the crystalline lens.

3. Implant according to one of claims 1 or 2, **characterised in that** the optical element (10) forms a multifocal lens with a base power of -10 to +10 diopters in order to correct the residual ametropia and an additional power for close vision of between +2 and +5 diopters.

4. Implant forming a multifocal intraocular lens comprising an optical element (10) able to correct the residual ametropia resulting from a main implant and haptic elements (13), **characterised in that** the implant is conformed for implantation in the ciliary sulcus (5).

**Patentansprüche**

1. Intraokularimplantat-Anordnung für das vordere Augensegment, mit einer Hautpintraokularlinse (2), die ein optisches Element und haptische Elemente, die sich zur Peripherie des optischen Elements erstrecken, umfasst, wobei die Hautpintraokularlinse für die Implantation in den Kapselsack (4) geeignet ist, und mit einer Sekundärlinse (1), die haptische Elemente (13) und ein optisches Element (10), das beschaffen ist, um eine Korrektur einer aus der Hauptlinse resultierenden Restametropie zu bewirken, umfasst, wobei das optische Element der sekundären Intraokularlinse multifokal (11a, 11b) ist, um die Nahsicht zu korrigieren, **dadurch gekennzeichnet, dass** die sekundäre Intraokularlinse (1) für die Implantation in den Ziliensulkus (5) geeignet ist.

2. Implantatanordnung nach Anspruch 1, **dadurch gekennzeichnet, dass** die Hauptintraokularlinse (2) monofokal (2) ist und beschaffen ist, um die Augenlinse optisch zu ersetzen.

3. Implantat nach einem der Ansprüche 1 oder 2, **dadurch gekennzeichnet, dass** das optische Element (10) eine multifokale Linse mit einer Basisstärke von -10 bis +10 Dioptrien, um die Restametropie zu korrigieren, und einer Zusatzstärke für die Nahsicht im Bereich von +2 bis +5 Dioptrien bildet.

4. Implantat, das eine multifokale Intraokularlinse bildet und ein optisches Element (10), das die aus einem Hauptimplantat resultierende Restametropie korrigieren kann, und haptische Elemente (13) umfasst, **dadurch gekennzeichnet, dass** das Implantat für die Implantation in den Ziliensulkus (5) geeignet ist.

*FIG.1*

*FIG.2*

*FIG.3*

**EP 1 200 020 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5769890 A

- WO 9407435 A